Europäisches Patentamt

European Patent Office

Office européen des brevets

(11) Publication number: **0 476 178 A1**

# EUROPEAN PATENT APPLICATION

(21) Application number: **90118186.7**

(22) Date of filing: **21.09.90**

(51) Int. Cl.⁵: **A61B 17/12**

(43) Date of publication of application:
**25.03.92 Bulletin 92/13**

(84) Designated Contracting States:
**AT BE CH DE ES FR GB IT LI NL SE**

(71) Applicant: **BIOPLEX MEDICAL B.V.**
**Selzerbeeklaan 9**
**NL-6291 HV Vaals(NL)**

(72) Inventor: **Janzen, Ernst, Dipl.-Ing.**
**Standelkruid 8**

**1251 GR Laren(NL)**
Inventor: **Rüttgers, Günter, Dipl.-Ing.**
**Spinnereistr. 8**
**W-5190 Stolberg(DE)**

(74) Representative: **Brauns, Hans-Adolf, Dr. rer.**
**nat. et al**
**Hoffmann, Eitle & Partner, Patentanwälte**
**Arabellastrasse 4**
**W-8000 München 81(DE)**

(54) Device for placing styptic material on perforated blood vessels.

(57) The invention relates to a device for placing styptic material, in particular of fibrous or spongy form, on perforated blood vessels, in particular arteries. A tube arrangement is provided which consists at least of a plug holder (2) and a thin-walled sheath (1) connected therewith which serves to guide the styptic material onto a perforation of a blood vessel. The device also comprises a pusher (3) which is arranged to be longitudinally displaceable in the tube arrangement, and a tissue dilator (10) guided in the tube arrangement essentially free of play, the tissue dilator having an outer diameter greater than that of the perforation and a coaxial guide channel (11).

Fig. 1

The invention relates to a device for placing styptic material, particularly of a fibrous or spongy form on perforated blood vessels, in particular arteries.

In certain medical operations on the human body, such as cardiac catheterisation and dilatation, a catheter or other aid is inserted through the arteries up to the heart or the heart region of patients. For this, the groin region of the human body represents a suitable location for the insertion of such instruments into the artery, whereby the artery runs from 1.5 to 4 cm beneath the skin and can be easily located. In order to insert the instrument into the artery, this is pierced or perforated. However, after completing such an operation and removing the devices, significant problems of bleeding through the perforation in the artery often arise.

In order to stop the bleeding and avoid hematomas or aneurisms, it has been customary to apply pressure to the artery in the perforated region for a 1/2 to 1 hour or longer until the opening in the artery has closed to the extent that it resists the blood pressure and does not burst open again. The personnel costs for such an operation are therefore high, as skilled workers must concern themselves with the closing of the perforated artery for a long time after the actual operation. Furthermore, the potential danger of aneurisms or haematomas occurring on account of the bursting open of the arteries at the weakened point exists for a long time after the operation.

A large loss of blood often arises if the artery bursts and can only be compensated for by blood transfusions. Additionally, in such a case, the perforation must often be repaired by means of a further operation.

DE-PS 30 20 611 describes a collagen fleece. Such a fleece would be applied to a defect of an artery to stop bleeding by haemostasis in order to prevent haematoma or an aneurism. However, problems arise with the exact positioning of the collegenic fleece onto the perforation of the artery, which is embedded in the tissue. When the collagenic fleece does not cover the entire perforation or only covers this with an edge portion thereof, the further danger exists that the perforation could open again at this weak-point on account of blood pressure. Consequently, the patient must still be taken care of by skilled medical personnel and pressure be applied to the blood vessel for a long period of time in order to remove the risk of an haematoma.

In accordance with EP-0367516 one has also attempted to provide a device and a method for closing and sealing off a perforation in an artery. After the catheter of the artery is removed, a cannula is placed in the tissue over the perforation and a tube having an inflatable balloon is supplied through the cannula over the perforation in the artery. A mechanical pressure can be applied on the perforation in the artery by inflating the balloon through the tube and the perforation is welded by a laser fed into the tube. However, it is questionable whether such a device can exactly locate the perforated region with the cannula after the catheter has been pulled out and whether it is ensured that the balloon is placed about the artery rather than displacing soft tissue about the perforation.

It is therefore an object of the invention to provide a device for placing styptic material on perforations in blood vessels with which a simple, quick and precise covering of the perforation in the blood vessel can be ensured.

This object is solved in accordance with the present invention by the features of claim 1.

After a cannula located in the perforation is pulled out, this cannula being used for inserting probes into the artery, a guide wire remains in the artery which projects out from the perforation. Subsequently, a tissue dilator blunt at its front end is pushed along the guide wire via its guiding channel until it lies over the perforation. The tubing arrangement, which essentially has no play and is guided along the tissue dilator, is then also pushed up to the perforation. The tubing arrangement is then held in position and the tissue dilator together with the guide wire are then pulled away from the perforation out of the tubing arrangement. As pressure is always applied to the artery during this operation and no blood pressure is present, the perforation is contracted to such an extent that the danger of entering the artery with the sheath of the tubing arrangement does not arise. In addition, the diameter of the sheath and the outer diameter of the plug respectively are considerably greater than the diameter of the perforation.

The very thin-walled sheath is supported and guided into the groin tissue by the tissue dilator along the guide wire so that it is not deformed.

A prepared plug (preferably made of a collagenic material in the form of a collagenic fleece or a collagenic sponge, which represent all styptic materials here) located in the plug holder can then be pushed through the tube arrangement with the pusher, during which the front end of the sheath continues to lie on the perforation and is held in this position. With such an arrangement, a plug can be precisely positioned to the outer side of an artery wall perforated by an operation thereon in order to stop bleeding by haemostasis and thus prevent haematoma or an aneurism. The great amount of time required for the conventional method of applying pressure to the artery can also be considerably reduced. The artery is simply covered over from the outside so that blockages and other dangers such as clots can be avoided.

In a preferred embodiment of the device, the plug holder and the sheath have an essentially similar and constant inner diameter along their length. This ensures that the plug can be pushed from the plug holder into the sheath without transition.

In accordance with a further embodiment of the device, the sheath and the plug holder are arranged coaxially in series.

According to a further useful embodiment, the plug holder is joined with the sheath at an acute angle of approximately 5 to 45°. In this way, the tissue dilator can be pushed through the tissue up to the perforation together with the sheath which has been slid thereover. The tissue dilator and the guide wire can then be removed together from the tube system and the plug located in the plug holder can be applied onto the perforation through the plug holder and the sheath by means of the flexible pusher.

In accordance with a further embodiment of the invention, the tube arrangement consists of a tube acting as both a thin-walled sheath and as a plug holder, a guide tube for the tissue dilator being connected with the tube at an acute angle. In this case, the flexible tissue dilator and the guide wire can be removed from the sheath and out of the guide tube, after which the plug in the plug holder can be applied onto the perforation with the pusher.

In one advantageous embodiment, the sheath is connected to the plug holder by a screw fitting or a plug connection. A further preferred embodiment provides for an integral sheath and plug holder. Thus, the inconvenient connection of the device is spared and the number of parts can also be minimized.

The pusher has a stop at its free rear end in accordance with an advantageous embodiment of the invention, the stop being movable up to the free end of the plug holder and having a surface for the thumb for the application pressure. In a useful embodiment, in order to maintain the tube arrangement in position against the pushing pressure, a finger support provided on the outer side of the tube arrangement, in particular the plug holder, is advantageous.

In accordance with a further particular embodiment of the inventive device, the length through which the pusher may be freely pushed essentially corresponds to the entire length of the tube arrangement. This ensures that the pusher presses the collagenic plug out of the tube arrangement but not through the perforation in the artery. Furthermore, the outer diameter of at least the free end of the pusher, apart from the play, corresponds to the inner diameter of the tube arrangement. Thus, a free displacement of the pusher can be ensured.

So as not to unnecessarily enlarge the opening of the human tissue at the artery, it is advantageous to have the sheath thin-walled and select an inner diameter of the tube system or the outer diameter of the plug which is 10 to 30% larger than the diameter of the artery perforation. For a catheter having a measurement of 8 French, the sheath has at least 11 French.

Preferably, styptic material in the form of a collagenic plug having its end facing the perforation consisting of loosened collagenic fleece is placed in the plug holder. The collagenic plug can also be a sponge.

In a further preferred embodiment, the device consists of sterilisable material, the parts of which are preferably sterile and packed as disposable parts. In this case, the styptic material is already placed in the plug holder so that the device can be used directly after being unpacked.

Three particularly advantageous exemplified embodiments described in the following are shown in the drawings, in which:

Fig. 1     shows a tube arrangement according to the invention with a separate plug holder and a linearly guided tissue dilator;

Fig. 2     shows a tube arrangement according to the invention with a bent tissue dilator; and

Fig. 3     shows a tube arrangement of the invention with a sheath and a plug holder arranged coaxially in series.

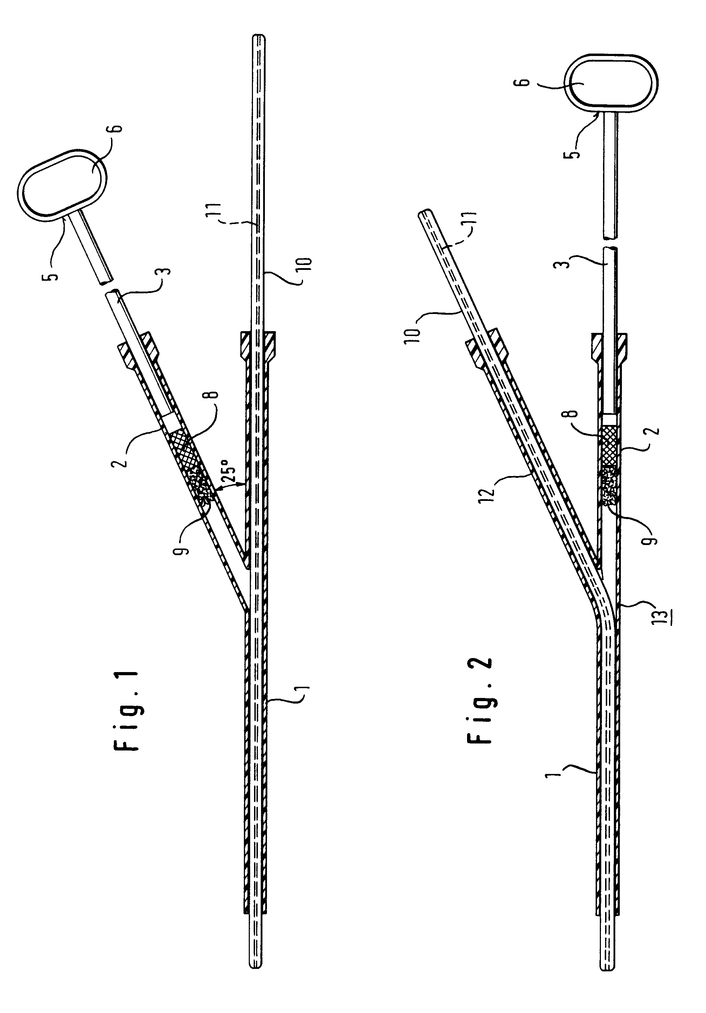

The device depicted in Fig. 1 shows a sheath 1 integrally joined to a plug holder 2 at an acute angle of approximately 25°. A tissue dilator 10 having a guide channel 11 extending along its entire length is arranged in the sheath 1 to be displaceable in the longitudinal direction. A styptic material in the form of a collagenic plug 8 having its end facing the perforation of loosened collagenic fleece 9 is arranged in the plug holder 2. The plug 8, 9 can be pushed directly through the plug holder 2 into the sheath 1 and onto the perforation by means of a flexible pusher 3, which is also arranged to be displaceable in the longitudinal direction in the plug holder 2, after the tissue dilator 10 and the guide wire have been removed.

Fig. 2 shows a tube 13 which serves both as a thin-walled sheath 1 and as a plug holder 2, a guide tube 12 for the tissue dilator 10 integrally formed with the tube 13 being connected to the tube 13 at an acute angle. The tissue dilator extends through the guide tube 12 and the sheath 1 and is withdrawn out of the tube arrangement together with the guide wire after the sheath 1 is placed on the perforation. The plug 8, 9 already placed in the plug holder can be pushed onto the perforation through the plug holder 2 and the sheath 1 by means of the pusher 3 also located in the plug holder 2. The rearward free end of the pusher 3 includes a stop 5, which can be moved against the free end of the plug holder, and also has a pressure application surface 6 for the thumb.

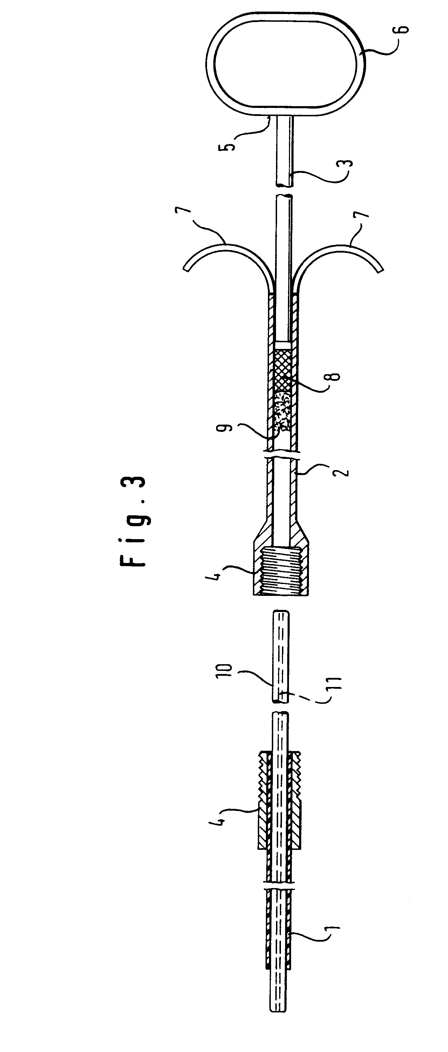

The device depicted in Fig. 3 shows a thin-walled sheath 1 in which a dilator 10 is arranged. A plug holder 2 includes finger supports 7 which maintain the tube arrangement in position against the pressure of the pusher 3. The pusher 3 has a stop 5, which may be brought into contact with the plug holder 2, and also includes a pressure application surface 6 for the thumb. A collagenic plug 8, 9 and the pusher 3 are inserted in the plug holder 2. Also, the free end of the sheath 1 and the plug holder 2 respectively consist of an outer and an inner thread 4.

When the sheath 1 has been placed on the perforation via the tissue dilator 10 and the latter located in the sheath 1 is removed out of the sheath together with the guide wire, the plug holder 2 comprising the plug of collagenic material is screwed onto the sheath 1. The pusher 3 is held with the thumb placed on the pressure application surface 6 and with the index and middle fingers on the finger supports 7. Subsequently, the collagenic plug 8, 9 is pushed with the pusher 3 through the tube arrangement to the end of the sheath 1 which lies on the perforation of the artery so that the styptic material 8, 9 leaves the arrangement. Thereafter, pressure is applied onto the styptic material with the device for approximately 5 minutes before it is removed through the puncture channel in the tissue. After the first minute, the pressure applied on the artery is reduced in order to allow the blood to circulate. The haemostatic effect of the plug is thus activated and strengthened by the fresh blood flowing by the perforation.

Thus, it may be seen that a precise placement of styptic material onto perforations in blood vessels can be carried out with this device, whereby the danger of haematomas or aneurisms is avoided and the long time required in the conventional methods for the application of pressure on the artery also lapses.

**Claims**

1. Device for placing styptic material, in particular of a fibrous or spongelike form, onto perforated blood vessels, in particular arteries, characterized by
   - a tube arrangement consisting at least of a plug holder (2) and a thin-walled sheath (1) connected therewith for guiding the styptic material to a perforation in a blood vessel,
   - a pusher (3) insertable into the tube arrangement to be displaceable in the longitudinal direction, and
   - a tissue dilator (10) guidable in the tube arrangement essentially without play, the tissue dilator (10) having an outer diameter greater than that of the perforation, and a coaxial guiding channel (11).

2. Device according to claim 1, characterized in that the plug holder (2) and the sheath (1) have an essentially similar inner diameter constant over their entire length.

3. Device according to claim 1 and 2, characterized in that the sheath (1) and the plug holder (2) are arranged coaxially in series.

4. Device according to claim 1 and 2, characterized in that the plug holder (2) is connected to the sheath (1) at an acute angle of approximately 5 to 45°.

5. Device according to claim 1, characterized in that the tube arrangement has a tube (13) serving as a thin-walled sheath and as a plug holder (2), a guide tube (12) for the tissue dilator (10) being connected to the tube (13) at an acute angle.

6. Device according to one of the preceding claims, characterized in that the sheath (1) is connected to the plug holder (2) by means of a screw fitting (4) or a plug connection.

7. Device according to claims 2 to 5, characterized in that the sheath (1) is integrally formed with the plug holder (2).

8. Device according to one of the preceding claims, characterized in that the pusher (3) has a stop (5) arranged at its free rear end, the stop being movable against the free end of the plug holder (2) and having a pressure application surface (6) for the thumb.

9. Device according to one of the preceding claims, characterized in that the freely displaceable length of the pusher (3) corresponds at least to the entire length of its guiding channel.

10. Device according to one of the preceding claims, characterized in that the outer diameter of the free end of the pusher (3) guided in the tube arrangement (2) corresponds essentially to the inner diameter of the tube arrangement.

11. Device according to one of the preceding claims, characterized in that finger supports (7) are provided on the outer side of the tube arrangement in order to maintain the tube arrangement in position against the pushing pressure.

12. Device according to one of the preceding claims, characterized in that styptic material in the form of a collagenic plug (8) having its end facing the perforation of loosened collagenic fleece (9) is placed in the plug holder (2).

13. Device according to one of the preceding claims, characterized in that the tube holder (2) is transparent.

14. Device according to one of the preceding claims, characterized in that the inner diameter of the tube arrangement and the outer diameter of the plug are respectively approximately 10 - 30% greater than the diameter of the artery perforation.

15. Device according to one of the preceding claims, characterized in that the device is formed of sterilisable material.

16. Device according to one of the preceding claims, characterized in that all parts of the device are packed sterile and air-tight as disposable parts.

EP 0 476 178 A1

# Fig. 1

# Fig. 2

Fig.3

EP 0 476 178 A1

European
Patent Office

EUROPEAN SEARCH
REPORT

Application Number

EP 90 11 8186

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | WO-A-8 911 301 (KENSEY NASH)<br>* Page 5, lines 28-34; page 10, lines 28-29; figures 1-3,5 *<br>– – – | 1-16 | A 61 B 17/12 |
| A | DE-U-8 907 370 (P. DIMITROV)<br>* Figure 2; page 6, lines 10-14 *<br>– – – | 4,5 | |
| A | US-A-4 895 564 (FARREL)<br>– – – | | |
| A | FR-A-2 641 692 (NIPPON ZEON)<br>– – – | | |
| A | US-A-4 744 364 (KENSEY)<br>– – – – – | | |

TECHNICAL FIELDS
SEARCHED (Int. Cl.5)

A 61 B
A 61 M

The present search report has been drawn up for all claims

| Place of search | Date of completion of search | Examiner |
|---|---|---|
| The Hague | 06 May 91 | BARTON S.A. |

CATEGORY OF CITED DOCUMENTS

X : particularly relevant if taken alone
Y : particularly relevant if combined with another
document of the same catagory
A : technological background
O : non-written disclosure
P : intermediate document
T : theory or principle underlying the invention

E : earlier patent document, but published on, or after
the filing date
D : document cited in the application
L : document cited for other reasons

& : member of the same patent family, corresponding
document